# EUROPEAN PATENT APPLICATION

(11) **EP 4 660 604 A1**
(43) Date of publication of application: **10.12.2025**
(21) Application number: 25157678.1
(22) Date of filing: 13.02.2025
(51) Int. Cl.: G01N 3/08, G01N 33/38, G01N 3/40

(54) **STRENGTH DETECTION DEVICE AND METHOD FOR CERAMIC PROCESSING**

(30) Priority: 06.06.2024 CN 202410729527
(71) Applicant: Shenzhen Yurucheng Dental Materials Co., Ltd., Shenzhen 518000 (CN)
(72) Inventor: Zongyu, LI, Shenzhen, 518000 (CN); LIU, Wei, Shenzhen, 518000 (CN); LIU, Jianjun, Shenzhen, 518000 (CN); ZHANG, Yu, Shenzhen, 518000 (CN); YANG, Qing, Shenzhen, 518000 (CN)
(74) Representative: Monteiro Alves, Inês

(57) **Abstract**

Disclosed are a strength detection device and method for ceramic machining. The strength detection device includes a self-rotating turntable assembly, a feeding mechanism and a strength detection mechanism. The feeding mechanism is provided above the accommodating groove and is configured to feed the ceramic workpiece into the accommodating groove. The strength detection mechanism includes a lifting assembly and a visual assembly. The driving end of the lifting assembly is connected with a pressing assembly, the pressing assembly includes a telescopic pressing head, the telescopic pressing head is sleeved with a spring, and one end of the spring is connected with a force sensing element configured to detect a force value. The visual assembly is provided on one side of the pressing assembly and is configured to detect the appearance pattern of the ceramic workpiece when the ceramic workpiece is pressed by the pressing assembly.

## Description

### TECHNICAL FIELD

The present application relates to the technical field of strength detection, and in particular to a strength detection device and method for ceramic processing.

### BACKGROUND

Ceramic materials are widely used in the fields of construction, decoration, industry, etc. because of their good chemical stability, high hardness and excellent wear resistance. With the development of modern industrial technology and the increase of market demands, the requirements on the quality and safety performance of ceramic products are also improved, and in particular, in the field of high-performance ceramics, such as industries of automobiles, electronic equipment and the like, more strict standards are provided for the strength and the durability of ceramic materials. Therefore, developing effective ceramic strength detection equipment and technology is important to ensure product quality and safety.

The existing ceramic strength detection technology mainly adopts a detection mode of a conveyor belt. A ceramic workpiece to be detected is arranged in a detection support and moves along with the conveyor belt, and a pressing mechanism is used for pressing and testing the ceramic at a detection station. The compressive strength of the ceramic is judged by observing whether the ceramic is cracked or detecting the cracking sound audibly. However, there are several disadvantages to this approach: firstly, the mechanical behavior in the pressing process often lacks accurate quantification, so that the accuracy of a test result is limited; secondly, only whether cracks appear or not is used as a judging standard, and microstructural changes or micro-cracks of the ceramic before the cracks do not appear are ignored, so that the comprehensiveness and accuracy of evaluation are affected; finally, the press test is destructive, and once too much pressure is applied, it is easy to cause damage to a good workpiece, increasing material waste and production costs.

In view of this, improvements are needed to the existing ceramic strength detection technology to solve the technical problem of limited compressive strength detection accuracy.

### SUMMARY

The objective of the present application is to provide a strength detection device and method for ceramic processing to solve the above technical problems.

To achieve this objective, the present application adopts the following technical solutions:
A strength detection device for ceramic processing includes:
a self-rotating turntable assembly provided with a plurality of accommodating grooves for receiving a ceramic workpiece in a peripheral direction of the turntable assembly, wherein each accommodating groove is correspondingly provided with a clamping assembly;
a feeding mechanism and a strength detection mechanism sequentially provided in a circumferential direction of the turntable assembly, wherein the feeding mechanism is provided above the accommodating groove and is configured to feed the ceramic workpiece into the accommodating groove;
the strength detection mechanism includes a lifting assembly and a visual assembly; a driving end of the lifting assembly is connected with a pressing assembly, the pressing assembly includes a telescopic pressing head sleeved with a spring, and one end of the spring is connected with a force sensing element for detecting a force value; and the visual assembly is provided at one side of the pressing assembly and is configured to detect an appearance pattern of the ceramic workpiece when the ceramic workpiece is pressed by the pressing assembly.

In an embodiment, the feeding mechanism includes a bracket and a feeding rail; a rotating shaft is provided at an upper end of the bracket, an angle adjusting assembly is provided at the rotating shaft, and the feeding rail is mounted on the angle adjusting assembly;
one end of the feeding rail is provided with a limiting assembly, and a receiving assembly is provided below the limiting assembly; and a discharging channel is formed in a vertical direction of the receiving assembly, and a lower port of the discharging channel is provided corresponding to the accommodating groove.

In an embodiment, the limiting assembly includes a connection block provided on the feeding rail, and a driving cylinder mounted on the connection block;
the connection block is provided with a sliding groove in a length direction, and a limiting block is slidably connected in the sliding groove;
a piston rod of the driving cylinder is connected to the limiting block, and the driving cylinder is configured to drive the limiting block to extend into the feeding rail to limit the ceramic workpiece; and
the receiving assembly is provided with a guide groove communicated with the discharging channel, an inner side wall of the guide groove is provided with an arc-shaped guide surface, and one end of the guide surface is configured to face the discharging channel.

In an embodiment, the pressing assembly includes an adjusting cylinder, a piston rod of the adjusting cylinder is connected to one end of the telescopic pressing head, and the adjusting cylinder is configured to drive the telescopic pressing head to press the ceramic workpiece.

In an embodiment, a sorting mechanism is provided in the circumferential direction of the turntable assembly, the sorting mechanism includes a first discharging assembly for discharging qualified ceramics and a second discharging assembly for discharging no good (NG) ceramics, and the first discharging assembly and the second discharging assembly are respectively provided at different circumferential positions of the turntable assembly.

In an embodiment, the first discharging assembly includes a mounting seat, and a discharging guide rail is provided at the mounting seat;
the upper end of the mounting seat is provided with a pushing cylinder, the driving end of the pushing cylinder is provided with a discharging push block, and one side of the discharging push block close to the mounting seat is provided with a clamping groove; and
the pushing cylinder is configured to operate to drive the discharging push block to move outwards, and the clamping groove is configured to push the ceramic workpiece in the accommodating groove to be discharged into the discharging guide rail.

In an embodiment, the strength detection device for ceramic processing further includes a workbench, a rotary driving assembly mounted on the workbench; the rotary driving assembly includes a driving motor, an output shaft of the driving motor is provided with a divider, and the divider is connected to the turntable assembly;
a limiting plate is provided outside the turntable assembly, a lower end face of the limiting plate is provided with a supporting column, and one end of the supporting column is fixedly connected to the workbench; the turntable assembly is provided with a first notch part corresponding to the first discharging assembly, and a second notch part corresponding to the second discharging assembly; and
the supporting column is provided with a supporting plate, the supporting plate is provided below the accommodating groove, and a conveying rail for moving the ceramic workpiece is formed on an upper end face of the supporting plate.

In an embodiment, the turntable assembly is provided with a position detector in the circumferential direction, and the position detector is provided behind the sorting mechanism in a rotational direction of the turntable assembly and is configured to detect whether or not a ceramic workpiece is present in the accommodating groove passing through the position detector.

The present application further provides a strength detection method for ceramic processing, which is implemented by using the above-mentioned strength detection device for ceramic processing, and the method includes:
coating a detection coating on a ceramic workpiece in advance, and feeding, via a feeding mechanism, the ceramic workpiece coated with the detection coating into an accommodating groove;
driving, via a turntable assembly, the ceramic workpiece to rotate below a strength detection mechanism, driving, via a lifting assembly, a pressing assembly to move downwards to be abutted against the ceramic workpiece, and detecting, via a force sensing element, a force value;
driving the telescopic pressing head to continuously press down, so that the telescopic pressing head compresses the spring and generates pressure to act on the ceramic workpiece, capturing, via a visual assembly, an image sequence of the detection coating in real time during a pressing process, and detecting, via the force sensing element, a pressure value exerted on the ceramic workpiece; and
analyzing the image sequence of the detection coating, obtaining a deformation value of the ceramic workpiece, constructing a pressure value-deformation value curve graph based on the obtained pressure value and the obtained deformation value, and analyzing the pressure value-deformation value curve graph to obtain strength performance of the ceramic workpiece.

In an embodiment, the analyzing the image sequence of the detection coating, obtaining a deformation value of the ceramic workpiece, constructing the pressure value-deformation value curve graph based on the obtained pressure value and the obtained deformation value, and analyzing the pressure value-deformation value curve graph to obtain strength performance of the ceramic workpiece includes:
applying a digital image correlation (DIC) algorithm to the image sequence of the detection coating, and calculating displacement field and strain field of the ceramic workpiece at all moments from the image sequence, obtaining the deformation value, and performing denoising on the deformation value;
performing alignment processing on the obtained pressure values and obtained deformation values with respect to a time frame to enable the pressure value and the deformation value to be paired at the same time point, and respectively summarizing the pressure value and the deformation value obtained on a time axis to form a pressure value set and a deformation value set;
inputting the pressure value set and the deformation value set into chart software, and constructing the pressure value-deformation value curve graph according to a corresponding relation between the pressure value and the deformation value;
analyzing a linear stage and a nonlinear stage of the curve through the pressure value-deformation value curve graph, identifying a characteristic point of the ceramic workpiece, and evaluating elastic modulus and plastic deformation characteristics of the ceramic workpiece according to the characteristic point, wherein the characteristic point includes an elastic limit point, a yield point and a maximum strength point; and
comprehensively determining a mechanical performance index of the ceramic workpiece according to the characteristic point and the stage identified in the pressure value-deformation value curve graph to obtain the strength performance of the ceramic workpiece, wherein the mechanical performance index includes elastic limit, yield strength and fracture strength.

Compared with the related art, the present application has the following beneficial effects: during operation, the ceramic workpiece is fed into the accommodating groove through the feeding mechanism, the corresponding clamping assembly operates to clamp and position the ceramic workpiece, and the turntable assembly operates to drive the ceramic workpiece to rotate below the strength detection mechanism. The lifting assembly drives the pressing assembly to move downward to abut against the ceramic workpiece, and drives the telescopic pressing head to continue to press down, so that the telescopic pressing head compresses the spring and generates pressure on the ceramic workpiece. The spring transmits the force to the force sensing element to detect the current pressure value of the ceramic workpiece, combined with the visual assembly, the appearance pattern of the ceramic workpiece is detected when it is pressed with the corresponding pressure value, and combined with the pressure value-deformation value, the strength performance of the ceramic workpiece is determined. This device realizes strength detection on the automated production line through the cooperation of the feeding mechanism and the turntable assembly, thus improving production efficiency, and can accurately measure the force applied to the ceramic workpiece, obtaining the exact pressure-bearing capacity of the workpiece. The appearance pattern of the ceramic workpiece under pressure are recorded through the visual assembly, and combined with the deformation of the workpiece under different pressures, it is conducive to in-depth research on the strength performance of ceramics, more comprehensively reflect the true strength of the material, and improve detection accuracy.

### BRIEF DESCRIPTION OF THE DRAWINGS

In order to more clearly illustrate the embodiments of the present application or the technical solutions in the related art, the drawings required for use in the description of the embodiments or the related art will be briefly introduced below. Obviously, the drawings described below are only some embodiments of the present application. For persons skilled in the art, other drawings can be obtained based on these drawings without paying creative efforts.

The structures, proportions, sizes, etc. illustrated in the drawings of this specification are only used to match the contents disclosed in the specification so as to facilitate understanding and reading by persons skilled in the art. They are not used to limit the conditions under which the present application can be implemented, and therefore have no substantive technical significance. Any structural modification, change in proportion or adjustment of size, without affecting the effects and purposes that can be achieved by the present application, should still fall within the scope of the technical contents disclosed by the present application.
FIG. 1 is a schematic diagram of an overall structure of a strength detection device for ceramic processing according to a first embodiment of the present application.
FIG. 2 is a front view schematic structural diagram of the strength detection device for ceramic processing according to the first embodiment of the present application.
FIG. 3 is a schematic structural diagram of a feeding mechanism of the strength detection device for ceramic processing according to the first embodiment of the present application.
FIG. 4 is a schematic structural diagram of a feeding rail and a limiting assembly of the strength detection device for ceramic processing according to the first embodiment of the present application.
FIG. 5 is a schematic structural diagram of a strength detection mechanism of the strength detection device for ceramic processing according to the first embodiment of the present application.
FIG. 6 is a schematic structural diagram of a first discharging assembly of the strength detection device for ceramic processing according to the first embodiment of the present application.
FIG. 7 is a schematic structural diagram of a turntable assembly of the strength detection device for ceramic processing according to the first embodiment of the present application.

Description of reference signs: turntable assembly 10, accommodating groove 11, clamping assembly 12, limiting plate 18, supporting column 13, first notch part 14, second notch part 15, supporting plate 16, conveying rail 17, feeding mechanism 20, bracket 21, feeding rail 22, rotating shaft 23, angle adjusting assembly 24, limiting assembly 25, receiving assembly 26, discharging channel 261, connection block 251, driving cylinder 252, sliding groove 253, limiting block 254, guide groove 262, guide surface 263, strength detection mechanism 30, lifting assembly 31, visual assembly 32, pressing assembly 33, telescopic pressing head 331, spring 332, force sensing element 333, adjusting cylinder 334, sorting mechanism 40, first discharging assembly 41, second discharging assembly 42, mounting seat 411, discharging guide rail 412, pushing cylinder 413, discharging push block 414, clamping groove 415, workbench 50, rotary driving assembly 60, driving motor 61, divider 62, position detector 70.

### DETAILED DESCRIPTION OF THE EMBODIMENTS

In order to make the purpose, features and advantages of the present application more obvious and easy to understand, the technical solutions in the embodiments of the present application will be clearly and completely described below in conjunction with the drawings in the embodiments of the present application. Obviously, the embodiments described below are only part of the embodiments of the present application, not all of the embodiments. Based on the embodiments of the present application, all other embodiments obtained by persons skilled in the art without creative efforts are within the scope of the present application.

In the description of the present application, it should be understood that the terms "upper", "lower", "top", "bottom", "inside", "outside" and the like indicate positions or positional relationships based on the positions or positional relationships shown in the drawings, and are only for the convenience of describing the present application and simplifying the description, rather than indicating or implying that the device or element referred to must have a specific orientation, be constructed and operated in a specific orientation, and therefore cannot be understood as a limitation of the present application. It should be noted that when a component is considered to be "connected" to another component, it may be directly connected to the other component or there may be a centrally arranged component at the same time.

The technical solution of the present application is further described below with reference to the accompanying drawings and through specific implementation methods.

### First embodiment:

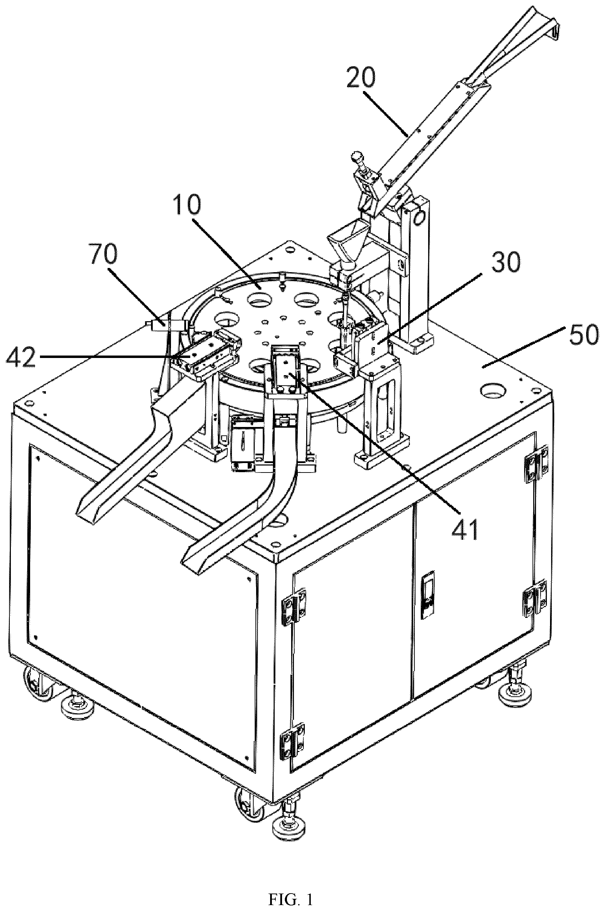

As shown in FIG. 1 to FIG. 7, an embodiment of the present application provides a strength detection device for ceramic processing, including a turntable assembly 10, the turntable assembly 10 is provided with a plurality of accommodating grooves 11 for accommodating ceramic workpieces in its peripheral direction, and each accommodating groove 11 is correspondingly provided with a clamping assembly 12. In the circumferential direction of the turntable assembly 10, a feeding mechanism 20 and a strength detection mechanism 30 are sequentially arranged. The feeding mechanism 20 is arranged above the accommodating groove 11, and is used for feeding the ceramic workpiece into the accommodating groove 11.

The strength detection mechanism 30 includes a lifting assembly 31 and a visual assembly 32. The driving end of the lifting assembly 31 is connected to a pressing assembly 33. The pressing assembly 33 includes a telescopic pressing head 331. A spring 332 is sleeved on the telescopic pressing head 331. One end of the spring 332 is connected to a force sensing element 333 for detecting the force value. The visual assembly 32 is arranged on one side of the pressing assembly 33 and is used to detect the appearance pattern of the ceramic workpiece when it is pressed by the pressing assembly 33.

The working principle of the present application is as follows: during operation, the ceramic workpiece is fed into the accommodating groove 11 through the feeding mechanism 20, the corresponding clamping assembly 12 operates to clamp and position the ceramic workpiece, and the turntable assembly 10 operates to drive the ceramic workpiece to rotate below the strength detection mechanism 30. The lifting assembly 31 drives the pressing assembly 33 to move downward to abut against the ceramic workpiece, and drives the telescopic pressing head 331 to continue to press down, so that the telescopic pressing head 331 compresses the spring 332 and generates pressure on the ceramic workpiece. The spring 332 transmits the force to the force sensing element 333 to detect the current pressure value of the ceramic workpiece, combined with the visual assembly 32, the appearance pattern of the ceramic workpiece is detected when it is pressed with the corresponding pressure value, and combined with the pressure value-deformation value, the strength performance of the ceramic workpiece is determined. Compared with the ceramic strength detection technology in the related art, this device realizes strength detection on the automated production line through the cooperation of the feeding mechanism 20 and the turntable assembly 10, thus improving production efficiency, and can accurately measure the force applied to the ceramic workpiece, obtaining the exact pressure-bearing capacity of the workpiece. The appearance pattern of the ceramic workpiece under pressure are recorded through the visual assembly 32, and combined with the deformation of the workpiece under different pressures, it is conducive to in-depth research on the strength performance of ceramics, more comprehensively reflect the true strength of the material, and improve detection accuracy.

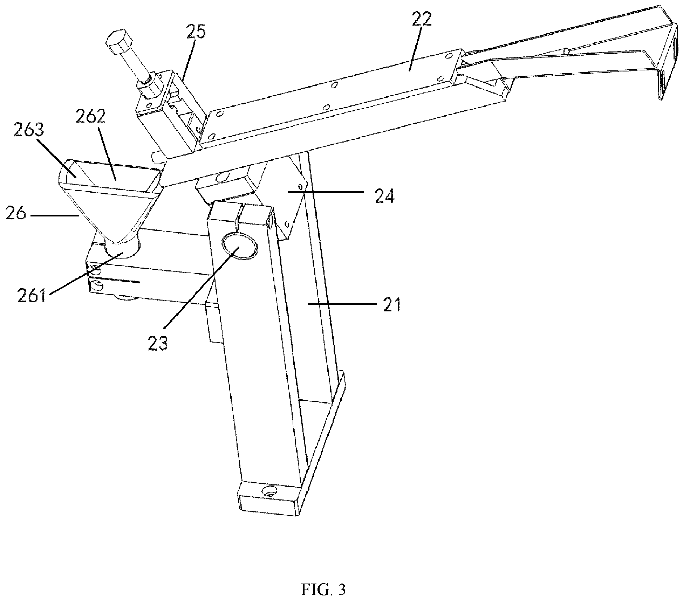

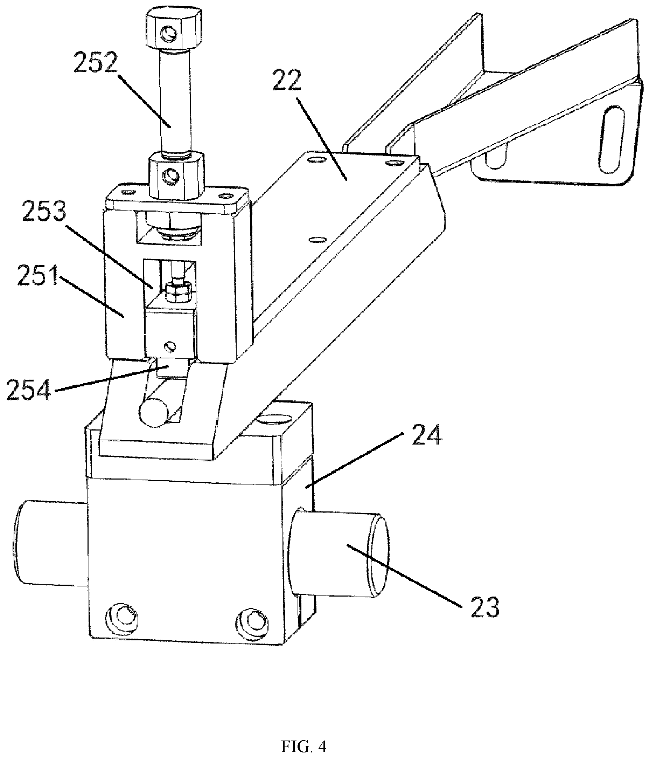

In this embodiment, as shown in FIG. 3 and FIG. 4, the feeding mechanism 20 includes a bracket 21 and a feeding rail 22. A rotating shaft 23 is provided at the upper end of the bracket 21, an angle adjusting assembly 24 is provided on the rotating shaft 23, and the feeding rail 22 is installed on the angle adjusting assembly 24. The rotating shaft 23 combined with the angle adjusting assembly 24 is used to fine-tune the inclination angle of the feeding rail 22, thereby controlling the speed and fluidity of the ceramic workpiece sliding along the rail to adapt to workpieces of different shapes and sizes.

A limiting assembly 25 is disposed at one end of the feeding rail 22, and a receiving assembly 26 is disposed below the limiting assembly 25. The receiving assembly 26 is provided with a discharging channel 261 in the vertical direction, and a lower end of the discharging channel 261 is disposed corresponding to the accommodating groove 11.

During operation, the ceramic workpiece to be detected is continuously added to the feeding rail 22, the ceramic workpiece enters the discharging channel 261 of the receiving assembly 26 along the feeding rail 22, and enters the accommodating groove 11 of the turntable assembly 10 after the ceramic workpiece is corrected in position through the discharging channel 261. The limiting assembly 25 and the discharging channel 261 ensure that the workpiece can enter the accommodating groove 11 in the correct direction and position, reducing the risk of incorrect positioning.

It should be noted that, depending on the type of workpiece or special requirements, it may be considered to introduce a vibration mechanism or an auxiliary propulsion device on the feeding rail 22 to ensure that the workpiece can move smoothly while avoiding damage to the ceramic workpiece.

It is further explained that the limiting assembly 25 includes a connection block 251 arranged on the feeding rail 22, and a driving cylinder 252 installed on the connection block 251. The connection block 251 is provided with a sliding groove 253 in its length direction, and a limiting block 254 is slidably connected in the sliding groove 253. The piston rod of the driving cylinder 252 is connected to the limiting block 254, and the driving cylinder 252 is used to drive the limiting block 254 to extend into the feeding rail 22 to limit the ceramic workpiece. The receiving assembly 26 is also provided with a guide groove 262 communicated with the discharging channel 261, the inner side wall of the guide groove 262 is provided with an arc-shaped guide surface 263, and one end of the guide surface 263 faces the discharging channel 261.

It should be noted that the limiting assembly 25 includes a connection block 251, a sliding groove 253 and a driving cylinder 252, which is intended to accurately control the position of the ceramic workpiece in the feeding rail 22. The limiting block 254 in the sliding groove 253 on the connection block 251 can be extended or retracted by the driving cylinder 252, thereby realizing the limiting effect on the ceramic workpiece moving along the rail. This design ensures that the ceramic workpiece can enter the discharging channel 261 of the receiving assembly 26 in an orderly manner to avoid material blockage. The guide groove 262 and the arc-shaped guide surface 263 provided in the receiving assembly 26 are responsible for guiding the ceramic workpiece to correctly enter the discharging channel 261, further improving the accuracy and smoothness of feeding.

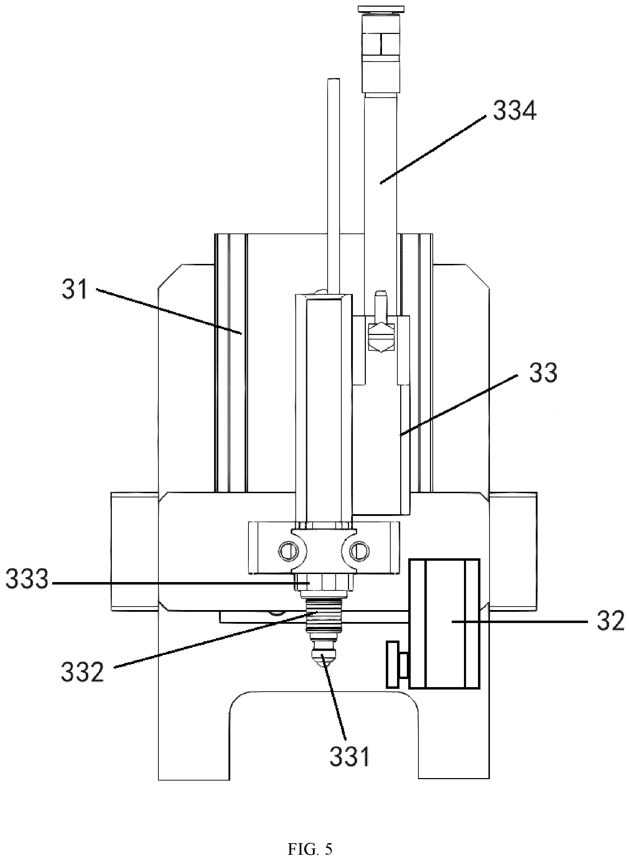

As shown in FIG. 5, the pressing assembly 33 further includes an adjusting cylinder 334, the piston rod of the adjusting cylinder 334 is connected to one end of the telescopic pressing head 331, and the adjusting cylinder 334 is used to drive the telescopic pressing head 331 to press the ceramic workpiece.

The adjusting cylinder 334 in the pressing assembly 33 is used to realize the secondary detailed adjustment of the telescopic pressing head 331. The lifting assembly 31 is used to complete the initial pressing, and the adjusting cylinder 334 provides more precise control, allowing the telescopic pressing head 331 to be further pressed down with a smaller displacement, which makes the pressure application more precise, and helps to improve the quality of pressing and improve the accuracy of strength detection. This design increases the flexibility of the detection device, and can closely cooperate with the lifting assembly 31 to meet the detection needs of different ceramic workpieces.

In this embodiment, a sorting mechanism 40 is further provided in the circumferential direction of the turntable assembly 10. The sorting mechanism 40 includes a first discharging assembly 41 for discharging qualified ceramics and a second discharging assembly 42 for discharging no good (NG) ceramics. The first discharging assembly 41 and the second discharging assembly 42 are respectively arranged at different circumferential positions of the turntable assembly 10.

It should be noted that the turntable assembly 10 is provided with a sorting mechanism 40, which includes the first discharging assembly 41 and the second discharging assembly 42, which are responsible for sorting qualified products and NG products respectively. This arrangement enables the turntable assembly 10 to automatically classify the detection results into qualified and unqualified categories after completing the strength detection, and send the workpieces to different discharging areas accordingly, which not only optimizes the automation level of the production process, but also greatly improves the production efficiency and the accuracy of quality control, and avoids mixing unqualified products into the final product.

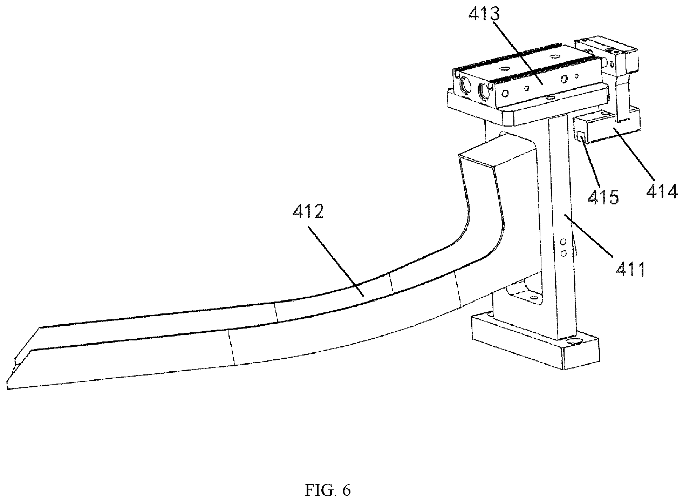

Specifically, as shown in FIG. 6, the first discharging assembly 41 includes a mounting seat 411, on which a discharging guide rail 412 is disposed. A pushing cylinder 413 is disposed at the upper end of the mounting seat 411, a discharging push block 414 is disposed at the driving end of the pushing cylinder 413, and a clamping groove 415 is disposed on the side of the discharging push block 414 close to the mounting seat 411. The pushing cylinder 413 operates to drive the discharging push block 414 to move outward, and pushes the ceramic workpiece in the accommodating groove 11 to discharge into the discharging guide rail 412 through the clamping groove 415, thereby realizing the discharging of the ceramic workpiece. It should be noted that in order to avoid damaging the ceramic workpiece during discharging, a layer of buffer pad can be laid in the discharging guide rail 412.

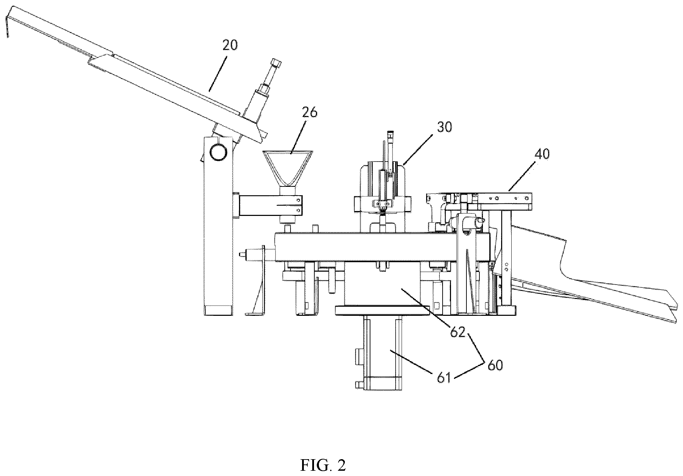

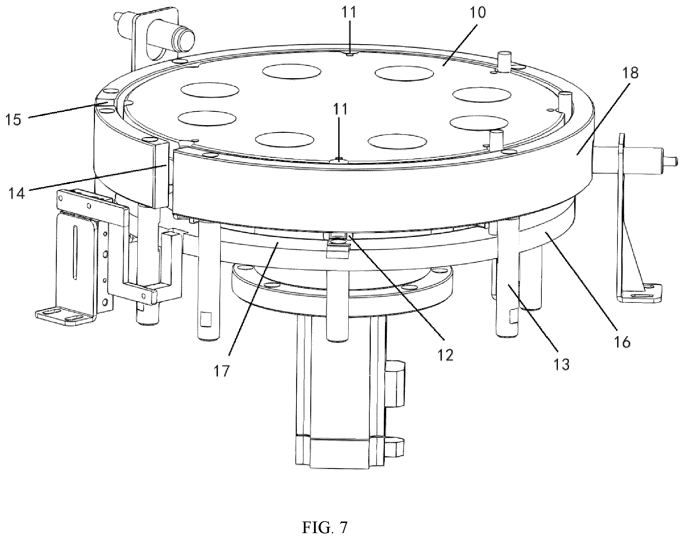

It is further explained that, in combination with FIG. 2 and FIG. 7, the strength detection device for ceramic processing further includes a workbench 50, on which a rotary driving assembly 60 is installed. The rotary driving assembly 60 includes a driving motor 61, the output shaft of the driving motor 61 is provided with a divider 62, and the divider 62 is connected to the turntable assembly 10. A limiting plate 18 is provided outside the turntable assembly 10, a supporting column 13 is provided on the lower end face of the limiting plate 18, and one end of the supporting column 13 is fixedly connected to the workbench 50. On the turntable assembly 10, a first notch part 14 is provided corresponding to the first discharging assembly 41, and a second notch part 15 is provided corresponding to the second discharging assembly 42. The supporting column 13 is provided with a supporting plate 16, the supporting plate 16 is provided below the accommodating groove 11, and the upper end face of the supporting plate 16 is formed with a conveying rail 17 for moving the ceramic workpiece. The first notch part 14 and the second notch part 15 correspond to the first discharging assembly 41 and second discharging assembly 42 respectively, so that the ceramic workpiece can be discharged smoothly when it reaches the corresponding position.

It should be noted that the strength detection device for ceramic processing incorporates the rotary driving assembly 60 and the divider 62 to drive the turntable assembly 10 to rotate precisely and achieve accurate positioning. With the limiting plate 18 and the supporting column 13, the turntable assembly 10 can maintain stable and reliable rotation, and the divider 62 provided on the workbench 50 ensures that the turntable performs strength detection on the accommodating grooves 11 one by one in the correct position. In addition, the combination of the supporting plate 16 and the conveying rail 17 not only supports the transfer of ceramic workpieces, but also provides stable support for ceramic workpieces during the strength detection process to prevent the pressed workpieces from falling.

In this embodiment, a position detector 70 is further provided in the circumferential direction of the turntable assembly 10. In the rotation direction of the turntable assembly 10, the position detector 70 is arranged behind the sorting mechanism 40 to detect whether there is a ceramic workpiece in the accommodating groove 11 passing through the position detector 70.

The position detector 70 is installed at the rear of the sorting mechanism 40 to confirm whether there are still ceramic workpieces in each accommodating groove 11 after passing through the sorting mechanism 40. This detection mechanism provides a quality control method to ensure the accuracy of the sorting process. Through real-time monitoring, the sorting process can be corrected in time to ensure the continuity of work and reduce downtime or quality problems caused by missed detection or false detection, thereby improving the automation and efficiency of the entire production line.

### Second embodiment:

The present application also provides a strength detection method for ceramic processing, which is implemented by using the strength detection device for ceramic processing as in the first embodiment. The strength detection method for ceramic processing includes:
coating a detection coating on a ceramic workpiece in advance, and feeding, via the feeding mechanism 20, the ceramic workpiece coated with the detection coating into the accommodating groove 11.

It should be noted that the detection coating is mainly prepared for subsequent digital image correlation (DIC) detection; the detection coating area on the workpiece surface needs to be evenly coated to ensure the accuracy of image analysis; the use of the feeding mechanism 20 realizes the automatic placement of the ceramic workpiece coated with the detection coating into the accommodating groove 11, thereby improving the efficiency and accuracy of feeding.

It should be understood that since the deformation value of ceramic workpieces is usually small, it is difficult for conventional cameras to detect the deformation area. Therefore, this solution adopts the DIC detection method, combined with the use of a detection coating, to detect tiny deformations of ceramic workpieces.
driving, via the turntable assembly 10, the ceramic workpiece to rotate below the strength detection mechanism 30, driving, via the lifting assembly 31, the pressing assembly 33 to move downwards to be abutted against the ceramic workpiece, and detecting, via the force sensing element 333, a force value; and
driving the telescopic pressing head 331 to continue pressing down, to enable the telescopic pressing head 331 to compress the spring 332 and generate pressure to act on the ceramic workpiece, capturing, via the vision assembly 32, an image sequence of the detection coating in real time during the pressing process, and detecting, via the force sensing element 333, a pressure value of the ceramic workpiece.

In the critical pressure application stage in deformation measurement, the workpiece is subjected to greater pressure by the continuously operating telescopic pressing head 331, and the compression of the spring 332 provides direct feedback of the force value change to the force sensing element 333. During the pressing process, the visual assembly 32 captures the image sequence of the detection coating on the ceramic workpiece in real time, so as to obtain deformation data through subsequent DIC analysis. This step must ensure the continuous and stable application of pressure and the capture of high-definition images to accurately measure the deformation of the ceramic workpiece under force.
analyzing the image sequence of the detection coating to obtain the deformation value of the ceramic workpiece, constructing a pressure value-deformation value curve graph based on the obtained pressure value and deformation value, and analyzing the curve graph to obtain the strength performance of the ceramic workpiece.

The deformation data are obtained by analyzing image sequence, and these data are combined with the pressure data recorded by the force sensing element 333 to construct a pressure value-deformation value curve graph. This curve graph plays a core role in evaluating the strength performance of ceramic workpieces. Through the curve graph, the reaction behavior of the material under different load conditions can be understood, and then the key performance parameters of ceramic workpieces such as elastic limit, yield strength and fracture strength can be accurately evaluated.

The beneficial effects of the present application are as follows: through the DIC detection composed of the visual assembly 32 and the pre-applied detection coating, a nondestructive detection method can be provided for the ceramic workpiece. The deformation of the workpiece surface under the load is captured and analyzed in real time by using the DIC technology, and the displacement and deformation of the pattern in the continuous image are evaluated by the algorithm to obtain the displacement field and strain field data of the whole field. Compared with the conventional mechanical test, this method can obtain more precise deformation data, which can not only realize the detection of the microstructure changes of the workpiece, but also avoid the loss of the workpiece that may be caused by destructive testing. At the same time, combined with the pressure value provided by the force sensing element 333, a pressure value-deformation value curve graph can be accurately constructed to achieve a more accurate evaluation of the strength performance of the ceramic workpiece. This method improves the precision of strength detection and provides more reliable technical support for ensuring the quality control and safety performance of ceramic workpieces.

In this embodiment, the analyzing the image sequence of the detection coating to obtain the deformation value of the ceramic workpiece, constructing the pressure value-deformation value curve graph based on the obtained pressure value and deformation value, and analyzing the curve graph to obtain the strength performance of the ceramic workpiece includes:
using a DIC algorithm on the image sequence of the detection coating, calculating the displacement field and strain field of the ceramic workpiece at each moment from the image sequence, obtaining a deformation value, and performing denoising on the deformation value.

The DIC algorithm is used to analyze the image sequence of the detection coating on the surface of the ceramic workpiece, from which the displacement field and strain field of the workpiece are calculated. These data provide quantitative values for the deformation of the workpiece at each moment of pressure. Denoising is performed to improve the accuracy of the analysis results and ensure that the deformation values obtained are not affected by noise and interference during the image capture process, thereby ensuring the reliability of the analysis data.

Performing alignment processing on the obtained pressure values and deformation values with respect to a time frame, so that the pressure values and the deformation values at the same time point are paired, and summarizing the pressure values and the deformation values obtained on the time axis respectively to form a pressure value set and a deformation value set.

The pressure value and the deformation value obtained by DIC analysis are aligned according to the time frame, so that each applied pressure value is paired with the deformation value at the same time point. The purpose of this alignment is to accurately reflect the deformation of the material under a specific pressure. The pressure value and deformation value set on the time axis are summarized to construct a pressure value-deformation value curve graph, which is the basic data set for subsequent analysis.

Constructing a pressure value-deformation value curve graph according to the corresponding relationship between the pressure value and the deformation value by inputting the pressure value set and the deformation value set into a chart software.

By inputting the processed pressure value set and deformation value set into the chart software, a curve graph is constructed according to the corresponding relationship between the pressure value and the deformation value. The pressure value-deformation value curve graph is a key tool for intuitively presenting the mechanical response of the material. Through it, the elastic and plastic behavior patterns of the material during the stress process can be observed.

Analyzing the linear stage and the nonlinear stage of the curve through the pressure value-deformation value curve graph, identifying the characteristic points of the ceramic workpiece, and evaluating the elastic modulus and plastic deformation characteristics thereof according to the characteristic points. The characteristic points include the elastic limit point, the yield point and the maximum strength point.

By observing the linear and nonlinear stages of the curve, various mechanical characteristic points of ceramic workpieces can be distinguished. The elastic limit point, yield point and maximum strength point are key reference points for evaluating the mechanical performance of materials. The elastic limit point indicates the maximum pressure that the material can withstand while its shape can be restored, the yield point indicates the initial pressure at which the material begins to undergo plastic deformation, and the maximum strength point represents the maximum pressure that the material can withstand before being destroyed.

It should be noted that in the initial linear stage, the slope of the curve can characterize the elastic modulus of the material. When the curve begins to deviate from the linearity and enters the nonlinear stage, it indicates that the material begins to enter the plastic deformation region. By analyzing the linear and nonlinear stages, the characteristic points of the elastic limit point, yield point and maximum strength point can be determined.

Comprehensively determining the mechanical performance indexes of the ceramic workpiece according to the characteristic points and stages identified in the curve graph to obtain the strength performance of the ceramic workpiece. The mechanical performance indexes include elastic limit, yield strength and fracture strength.

According to the identified curve characteristic points and curve stages, the mechanical performance indexes of ceramic workpieces, such as elastic limit, yield strength and fracture strength, are comprehensively evaluated, which are important parameters for measuring the overall performance of the workpiece. These indexes can not only determine the safety and practical application limits of the material in normal use, but also help optimize product design and material selection.

The above embodiments are only used to illustrate the technical solutions of the present application, rather than to limit the same. Although the present application has been described in detail with reference to the aforementioned embodiments, persons skilled in the art should understand that the technical solutions described in the aforementioned embodiments may still be modified, or some of the technical features may be replaced by equivalents. However, these modifications or replacements do not deviate the essence of the corresponding technical solutions from the spirit and scope of the technical solutions of the embodiments of the present application.

## Claims

1. A strength detection device for ceramic processing, **characterized by** comprising:
a self-rotating turntable assembly (10) provided with a plurality of accommodating grooves (11) for receiving a ceramic workpiece in a peripheral direction of the turntable assembly (10), wherein each accommodating groove (11) is correspondingly provided with a clamping assembly (12); and
a feeding mechanism (20) and a strength detection mechanism (30) sequentially provided in a circumferential direction of the turntable assembly (10),
wherein the feeding mechanism (20) is provided above the accommodating groove (11) and is configured to feed the ceramic workpiece into the accommodating groove (11);
the strength detection mechanism (30) comprises a lifting assembly (31) and a visual assembly (32);
a driving end of the lifting assembly (31) is connected with a pressing assembly (33), the pressing assembly (33) comprises a telescopic pressing head (331) sleeved with a spring (332), and one end of the spring (332) is connected with a force sensing element (333) for detecting a force value; and
the visual assembly (32) is provided at one side of the pressing assembly (33) and is configured to detect an appearance pattern of the ceramic workpiece when the ceramic workpiece is pressed by the pressing assembly (33).

2. The strength detection device for ceramic processing according to claim 1, wherein the feeding mechanism (20) comprises a bracket (21) and a feeding rail (22);
a rotating shaft (23) is provided at an upper end of the bracket (21), an angle adjusting assembly (24) is provided at the rotating shaft (23), and the feeding rail (22) is mounted on the angle adjusting assembly (24);
one end of the feeding rail (22) is provided with a limiting assembly (25), and a receiving assembly (26) is provided below the limiting assembly (25); and
a discharging channel (261) is formed in a vertical direction of the receiving assembly (26), and a lower port of the discharging channel (261) is provided corresponding to the accommodating groove (11).

3. The strength detection device for ceramic processing according to claim 2, wherein the limiting assembly (25) comprises a connection block (251) provided on the feeding rail (22), and a driving cylinder (252) mounted on the connection block (251);
the connection block (251) is provided with a sliding groove (253) in a length direction, and a limiting block (254) is slidably connected in the sliding groove (253);
a piston rod of the driving cylinder (252) is connected to the limiting block (254), and the driving cylinder (252) is configured to drive the limiting block (254) to extend into the feeding rail (22) to limit the ceramic workpiece; and
the receiving assembly (26) is provided with a guide groove (262) communicated with the discharging channel (261), an inner side wall of the guide groove (262) is provided with an arc-shaped guide surface (263), and one end of the guide surface (263) is configured to face the discharging channel (261).

4. The strength detection device for ceramic processing according to claim 1, wherein the pressing assembly (33) comprises an adjusting cylinder (334), a piston rod of the adjusting cylinder (334) is connected to one end of the telescopic pressing head (331), and the adjusting cylinder (334) is configured to drive the telescopic pressing head (331) to press the ceramic workpiece.

5. The strength detection device for ceramic processing according to claim 1, wherein a sorting mechanism (40) is provided in the circumferential direction of the turntable assembly (10), the sorting mechanism (40) comprises a first discharging assembly (41) for discharging qualified ceramics and a second discharging assembly (42) for discharging no good (NG) ceramics, and the first discharging assembly (41) and the second discharging assembly (42) are respectively provided at different circumferential positions of the turntable assembly (10).

6. The strength detection device for ceramic processing according to claim 5, wherein the first discharging assembly (41) comprises a mounting seat (411), and a discharging guide rail (412) is provided at the mounting seat (411);
the upper end of the mounting seat (411) is provided with a pushing cylinder (413), the driving end of the pushing cylinder (413) is provided with a discharging push block (414), and one side of the discharging push block (414) close to the mounting seat (411) is provided with a clamping groove (415); and
the pushing cylinder (413) is configured to operate to drive the discharging push block (414) to move outwards, and the clamping groove (415) is configured to push the ceramic workpiece in the accommodating groove (11) to be discharged into the discharging guide rail (412).

7. The strength detection device for ceramic processing according to claim 5, further comprising a workbench (50), wherein:
a rotary driving assembly (60) mounted on the workbench (50);
the rotary driving assembly (60) comprises a driving motor (61), an output shaft of the driving motor (61) is provided with a divider (62), and the divider (62) is connected to the turntable assembly (10);
a limiting plate (18) is provided outside the turntable assembly (10), a lower end face of the limiting plate (18) is provided with a supporting column (13), and one end of the supporting column (13) is fixedly connected to the workbench (50);
the turntable assembly (10) is provided with a first notch part (14) corresponding to the first discharging assembly (41), and a second notch part (15) corresponding to the second discharging assembly (42); and
the supporting column (13) is provided with a supporting plate (16), the supporting plate (16) is provided below the accommodating groove (11), and a conveying rail (17) for moving the ceramic workpiece is formed on an upper end face of the supporting plate (16).

8. The strength detection device for ceramic processing according to claim 5, wherein the turntable assembly (10) is provided with a position detector (70) in the circumferential direction, and the position detector (70) is provided behind the sorting mechanism (40) in a rotational direction of the turntable assembly (10) and is configured to detect whether or not a ceramic workpiece is present in the accommodating groove (11) passing through the position detector (70).

9. A strength detection method for ceramic processing, implemented using the strength detection device for ceramic processing according to any one of claims 1 to 8, **characterized by** comprising:
coating a detection coating on a ceramic workpiece in advance, and feeding, via a feeding mechanism (20), the ceramic workpiece coated with the detection coating into an accommodating groove (11);
driving, via a turntable assembly (10), the ceramic workpiece to rotate below a strength detection mechanism (30), driving, via a lifting assembly (31), a pressing assembly (33) to move downwards to be abutted against the ceramic workpiece, and detecting, via a force sensing element (333), a force value;
driving the telescopic pressing head (331) to continuously press down, so that the telescopic pressing head (331) compresses the spring (332) and generates pressure to act on the ceramic workpiece, capturing, via a visual assembly (32), an image sequence of the detection coating in real time during a pressing process, and detecting, via the force sensing element (333), a pressure value exerted on the ceramic workpiece; and
analyzing the image sequence of the detection coating, obtaining a deformation value of the ceramic workpiece, constructing a pressure value-deformation value curve graph based on the obtained pressure value and the obtained deformation value, and analyzing the pressure value-deformation value curve graph to obtain strength performance of the ceramic workpiece.

10. The strength detection method for ceramic processing according to claim 9, wherein the analyzing the image sequence of the detection coating, obtaining a deformation value of the ceramic workpiece, constructing the pressure value-deformation value curve graph based on the obtained pressure value and the obtained deformation value, and analyzing the pressure value-deformation value curve graph to obtain strength performance of the ceramic workpiece comprises:
applying a digital image correlation (DIC) algorithm to the image sequence of the detection coating, and calculating displacement field and strain field of the ceramic workpiece at all moments from the image sequence, obtaining the deformation value, and performing denoising on the deformation value;
performing alignment processing on the obtained pressure values and obtained deformation values with respect to a time frame to enable the pressure value and the deformation value to be paired at the same time point, and respectively summarizing the pressure value and the deformation value obtained on a time axis to form a pressure value set and a deformation value set;
inputting the pressure value set and the deformation value set into chart software, and constructing the pressure value-deformation value curve graph according to a corresponding relation between the pressure value and the deformation value;
analyzing a linear stage and a nonlinear stage of the curve through the pressure value-deformation value curve graph, identifying a characteristic point of the ceramic workpiece, and evaluating elastic modulus and plastic deformation characteristics of the ceramic workpiece according to the characteristic point, wherein the characteristic point comprises an elastic limit point, a yield point and a maximum strength point; and
comprehensively determining a mechanical performance index of the ceramic workpiece according to the characteristic point and the stage identified in the pressure value-deformation value curve graph to obtain the strength performance of the ceramic workpiece, wherein the mechanical performance index comprises elastic limit, yield strength and fracture strength.
